Europäisches Patentamt

European Patent Office

Office européen des brevets

(11) Publication number: **0 485 463 B1**

# EUROPEAN PATENT SPECIFICATION

(45) Date of publication of patent specification: **18.10.95**

(51) Int. Cl.⁶: **C12N 7/04**, C12N 7/08, A61K 39/23

(21) Application number: **90911906.7**

(22) Date of filing: **08.08.90**

(86) International application number: **PCT/AU90/00338**

(87) International publication number: **WO 91/02054 (21.02.91 91/05)**

(54) **ATTENUATED CANINE PARVOVIRUS (CPV), VACCINE COMPRISING CPV AND METHOD OF PREVENTING INFECTION BY CPV IN DOGS.**

(30) Priority: **08.08.89 AU 5670/89**

(43) Date of publication of application:
**20.05.92 Bulletin 92/21**

(45) Publication of the grant of the patent:
**18.10.95 Bulletin 95/42**

(84) Designated Contracting States:
**AT BE CH DE DK ES FR GB IT LI LU NL SE**

(56) References cited:
**EP-A- 0 117 767**
**WO-A-84/02847**
**AU-B- 5 405 679**
**AU-B- 7 155 381**
**AU-D- 5 280 186**

**CHEMICAL ABSTRACTS, vol. 96, no. 26, 1982, Columbus, OH (US); Microbiochemical Research Foundation, no. 223248**

(73) Proprietor: **ARTHUR WEBSTER PTY. LTD.**
**23 Victoria Avenue**
**Castle Hill, NSW 2154 (AU)**

(72) Inventor: **McGAVIN, David, Ross**
**4 Charles Street**
**Putney, NSW 2112 (AU)**
Inventor: **LAVIDIS, Lynette**
**6 Calandra Avenue**
**Ouakers Hill, NSW 2763 (AU)**

(74) Representative: **Perry, Robert Edward et al**
**GILL JENNINGS & EVERY**
**Broadgate House**
**7 Eldon Street**
**London EC2M 7LH (GB)**

JOURNAL OF THE AMERICAN VETERINARY MEDICAL ASSOCIATION, vol. 181, no. 9, 1982; pp. 909-913

DERWENT WPIL, Online Abstract; AN 88-041049

PATENT ABSTRACTS OF JAPAN, C-259; p. 124

DERWENT WPIL, Online Abstract; AN 87-123345

**Description**

Field of the Invention

Canine parvovirus (CPV-2) is a pathogen of dogs that is associated with vomiting, diarrhoea, depression, pyrexia, variable leukopenia and rapid dehydration. Parvovirus requires actively dividing host cells to support their growth and thus cells having a high turnover are most susceptible, and different tissues may be more susceptible at various stages of development. Accordingly, myocarditis and more importantly, enteritis are the two most common disease entities.

Background to the Invention

Puppies are especially susceptible to infection by the virus, particularly since the advent of regular vaccination regimes. Vaccinated bitches pass on a short lived immunity to the puppies as a result of the transfer of maternally derived antibody (MDA). Maternal antibodies provide some passive protection for puppies during their first weeks, the puppies often having a serum antibody level equal to 50% that of its parent. However, the maternally derived antibody declines exponentially with a half life of around about 8 - 10 days.

While the MDA continues to decline exponentially it has been found that vaccination during this period with commercially available vaccines may fail to elicit a response by way of boosted antibodies. This phenomenon is described in greater detail in various scientific papers, such as Cornell Veterinarian, 1982, 72: 103-119 (Pollock) and Cornell Veterinarian 1981, 71: 408-427 (Carmichael et al).

It appears that while MDA remains detectable by standard methods (such as haemagglutination - inhibition (HI), ELISA or serum neutralisation etc), vaccines are extremely unreliable in inducing an active immune response in the pup. MDA can be detected for some time after falling below a level that is considered to be a minimum to provide protection against disease (using the HI test this corresponds to a titre of approximately 64 Haemagglutination Inhibiting Units per 50 ul, or HIU/50uL). Therefore, there is a period of time (typically between 6-14 weeks of age) termed the "immunity gap", during which the puppy's maternally derived antibodies have fallen below what is considered protective and during which vaccination fails to elicit a response. During this period the puppy is susceptible to infection and is unresponsive to vaccination.

It has been postulated that a vaccine that is more antigenic than the prior art canine parvovirus vaccines would be capable of overcoming the maternally derived antibodies in puppies and stimulate their own immune system to produce protective antibodies.

This has been achieved to some extent with the use of less attenuated (and therefore more antigenic) strains of CPV-2, however, these strains have the serious disadvantage of being readily shed in the faeces of vaccinated animals thus posing a risk of spreading and potential reversion to virulence (see European patent specification No. 189958). An example of such a strain is CPV strain I-404 (Institute Pasteur) which is believed to be currently used in a commercial vaccine INTERVET "NOBIVAC PARVO-C".

The present inventors have surprisingly found through the selection of suitable strains that it is possible to produce a vaccine to protect against canine parvovirus disease which overcomes the particular problems of the prior art, including CPV strain I-404, relating to providing protection to young puppies that have detectable levels of circulating maternally derived antibodies and the problem of shedding large quantities of live virus in the faeces of vaccinated animals which can possibly infect other dogs.

Other problems with commercially available vaccines directed against parvovirus are documented. For instance, certain breeds are particularly susceptible to the immunity gap phenomenon. Also, some animals remain unresponsive to repeated doses of vaccine even after MDA levels would normally be expected to have almost disappeared.

Disclosures of the Invention

The present invention seeks to provide an alternative CPV-2 vaccine, which will elicit an antibody response in dogs in the presence of maternally derived anti CPV-2 antibodies and is of low virulence.

In a first aspect the present invention consists in an attenuated canine parvovirus having the antigenic and virulence characteristics of ECACC Accession No. V89042601 (CPV-2 P69).

In a preferred embodiment of the invention the parvovirus is such that any virus shed from dogs inoculated with the attenuated parvovirus has a low rate of infection.

As used in this specification the term "a low rate of infection" in relation to a parvovirus means that the parvovirus is such that it is not shed from infected animals or that if shed, the shed parvovirus is substantially incapable of infecting dogs which come into contact with the shed parvovirus.

In a preferred embodiment of the first aspect the present invention comprises a strain of canine parvovirus (CPV-2) that has been deposited by the present applicants on 26 April 1989 with the European Collection of Animal Cell Cultures (ECACC), Porton Down, Salisbury, U.K. and has been allocated the accession No. V89042601. This strain corresponds to passage 69 (P69) of an attenuated canine parvovirus strain K3i and hereinafter will be referred to as CPV-2 P69.

Attentuation of the virus culture for the purpose of the present invention has been accomplished by serial passage of the virus culture in a suitable culture system, such as feline kidney (FK) cell line. The virus is cloned using a limit dilution technique and passaged to give the viral seed lot designated passage 69 (P69).

In a second aspect the present invention consists in a vaccine comprising an effective amount of the attentuated canine parvovirus defined above.

In a preferred embodiment of the invention the attenuated canine parvovirus is such that any virus shed from dogs inoculated with the vaccine has a low rate of infection.

In a particularly preferred embodiment the second aspect of the invention consists in a vaccine comprising an effective amount of the CPV strain deposited with the ECACC under the accession number V89042601, corresponding to CPV-2 P69.

An effective amount, as referred to throughout the present description and claims, is an amount sufficient to elicit an immune response, preferably at least $10^{4.8}$ 50% Tissue Culture Infective Doses ($TCID_{50}$) per dose.

The vaccine of the present invention can utilize the novel CPV-2 P69 in either a live form or an inactivated killed form. Preferably, the vaccine of the present invention comprises live CPV-2 P 69 strain or its derivatives or functional equivalents.

The vaccine may comprise other constituents, such as stabilizers, adjuvants, excipients, other pharmaceutically acceptable compounds or any other antigen or part thereof. The vaccine may be in the form of a lyophilised preparation or as a suspension, all of which are common in the field of vaccine production.

In a preferred embodiment the vaccine of the present invention may be combined with other viruses or organisms that are used for vaccination (as is common in many current commercial vaccines) such as canine distemper, infectious hepatitis virus, canine parainfluenza or Bordetella, but not limited to these examples.

According to the present invention CPV-2 P 69 has been grown in a suitable cell culture system, preferably FK or canine kidney cells (such as MDCK), at optimal conditions and the culture is used in the formulation of a vaccine for administration, using well known techniques.

It has been found that CPV-2 P69 can be incorporated in a vaccine that is successful in providing protection against canine parvovirus disease, even in the presence of levels of maternally derived antibodies which would normally be expected to interfere with vaccination.

The present viral strain V89042601 is distinguishable from previously known strains of CPV-2 by virtue of its serological response in both seropositive and seronegative dogs, its growth characteristics in cell cultures, variations in its DNA sequence as well as by its particular shedding characteristics of live virus from animals administered a vaccine comprising the novel strain.

Particularly, the present invention may be distinguished from existing vaccine strains of CPV by a comparison of their viral yields when cell cultures are inoculated. There are obvious differences in the ability of various strains of CPV vaccine to grow in cell cultures of different types, for example feline versus canine cell cultures.

Antibody titres referred to throughout the present specification were measured using a standard haemagglutination-inhibition test using twofold dilutions and are expressed as the reciprocal of the highest serum dilution which completely inhibits agglutination of pig red blood cells.

Brief Description of the Drawings

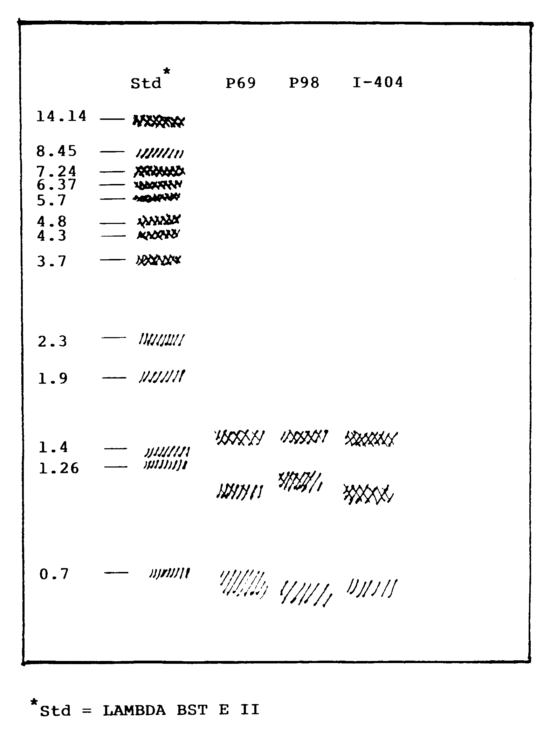

Fig. 1 - HpH-I digest of CPV-2 P69, CPV-2 P98 and CPV strain I-404 showing differences in the molecular weight of the different fragments.

Best Mode of Carrying out the Invention

1. Attenuation of CPV-2

A derivative of CPV-2 K3i strain isolated from a virulent case of parvovirus was obtained from James Cook University, Townsville, Australia. The virus was attenuated by serial passage in feline kidney (FK) sell line and dog kidney (MDCK) cell line by the applicant using standard passaging techniques.

The harvest of passage 61 was purified by standard limit dilution techniques. This purification method was repeated twice for a total of three limit dilution passages. The harvest of the third limit dilution passage (ie, passage 64) of the virus in FK cells was passaged a further time in a virus multiplication step (passage 65).

2. Preparation of Vaccine Seed Lots

Purified virus from passage 65 was passaged four times further by inoculation of FK cultures and incubated for three to four days each at 37ºC to give passage 69, which was stored at -70ºC and is the deposited strain designated V89042601 according to the preferred embodiment of the present invention.

3. Preparation of CPV-2 Vaccine comprising P 69

FK cell cultures are inoculated with P 68 and incubated at 37ºC for three to four days. The culture is harvested using known standard techniques (depending on whether a live or inactivated vaccine is desired) which gives a virus bulk which is then assayed for virus titre (potency) and sterility. The virus bulk is formulated together with a stabilizer and stored for use at -20ºC. The vaccine may be further processed by freeze drying if desired.

4A. Vaccination Results in Six Week Old Puppies

Six week old puppies (miniature fox terriers) were passively immunised with 2 ml of high titre positive serum via intra peritoneal injection. Pre vaccination (post passive immunization) titres were determined as shown in Table 1; antibody levels of ≤32 are not considered protective against the disease, however they can interfere with a puppy's response to the vaccination.

The puppies were then vaccinated with a vaccine comprising CPV-2 P 69 as described, utilising various dose regimes as indicated in Table 1. Antibody titres were measured at 15 days post vaccination and shown in Table 1. Only one dog, administered ($10^{4.1}$ TCID50) failed to respond.

TABLE 1

| Maternal Antibody Experiment | | |
|---|---|---|
| Vaccine Dose Administered (TCID50)) | Pre Vaccination Bleed (Post Passive Immunization) | 15 Days Post Vaccination (HIU/50uL) |
| $10^{4.1}$ | 32 | 8192-16384 |
| $10^{4.1}$ | 32 | 32-64 |
| $10^{5.1}$ | 16 | 8192 |
| $10^{5.1}$ | 32-64 | 16384 |
| $10^{5.1}$ | 16 | 8192 |
| $10^{6.1}$ | 16 | 32768 |
| $10^{6.1}$ | < 16 | 16384 |

4B. Comparison of Canine Parvovirus Vaccines in Seropositive Puppies

The serology data from seropositive pups six weeks old vaccinated with a vaccine containing either CPV-2 P69 or NOBIVAC PARVO-C batch 4545 (which is believed to contain CPV strain I-404) were compared.

5

Six week old beagle pups from bitches which were vaccinated with an inactivated CPV vaccine during pregnancy, were vaccinated with a single dose of the appropriate vaccine and revaccinated with the same vaccine at nine weeks of age.

The pups receiving NOBIVAC PARVO C were all litter mates of some of those pups receiving CPV-2 P 69, ie they were matched groups.

The vaccine including CPV-2 P69 was used at a titre of $10^{5.8}$ $TCID_{50}$ per dose. The Nobivac Parvo-C was used at a titre of $10^{5.4}$ $TCID_{50}$ per dose (this was the titre of Parvo-C as purchased).

Pups were bled at three, six, eight, nine and eleven weeks of age to measure antibody titres to CPV.

The titres of the bitches at whelping range from 32 to 1024 HIU/50uL. The pups at three weeks of age had maternally derived antibody levels against canine parvovirus which ranged from 16 to 512 HIU/50uL. These titres were approximately 50% of the dam. Haemagglutination inhibition titres of the pups before and after vaccination are given in Table 2.

EP 0 485 463 B1

**TABLE 2**
RESULTS OF SEROLOGY TESTS ON PUPS PRE AND POST VACCINATION

| VACCINE | PUP NO. | 3 | 6* | 8 | 9* | 11 |
|---|---|---|---|---|---|---|
| CPV-2 P69 | 9 | 512+ | 32 | 16384 | 4096 | 2048 |
| | 10 | 512 | 64 | 8192 | 2048 | 2048 |
| | 11 | 512 | 64 | 8192 | 8192 | 4096 |
| | 15 | 64 | <16 | 4096 | 4096 | 2048 |
| | 16 | 64 | <16 | 8192 | 4096 | 4096 |
| | 17 | 64 | <16 | 8192 | 2048 | 2048 |
| | 18 | 64 | <16 | 8192 | 2048 | 4096 |
| | 29 | 128 | 16 | 8192 | 4096 | 4096 |
| | 30 | 128 | 32 | 4096 | 4096 | 2048 |
| | 31 | 128 | 16 | 4096 | 2048 | 4096 |
| | 32 | 128 | 32 | 2048 | 2048 | 2048 |
| | 33 | 128 | 32 | 8192 | 4096 | 2048 |
| | 34 | 128 | 16 | 2048 | 4096 | 2048 |
| | 35 | 128 | 16 | 2048 | 4096 | 4096 |
| | 36 | 16 | <16 | 8192 | 2048 | 2048 |
| | 38 | 32 | <16 | 4096 | 2048 | 2048 |
| | 40 | 16 | <16 | 4096 | 2048 | 2048 |
| | 41 | 32 | <16 | 8192 | 2048 | 4096 |
| | 42 | 64 | <16 | 8192 | 4096 | 4096 |
| | 43 | 16 | <16 | 4096 | 2048 | 2048 |
| | 44 | 32 | <16 | 4096 | 2048 | 2048 |
| | 45 | 256 | 32 | 4096 | 2048 | 2048 |
| | 46 | 256 | 64 | 2048 | 4096 | 2048 |
| | 50 | 512 | 64 | 64 | 32 | 2048 |
| Nobivac Parvo-C | 12 | 256 | 32 | 16 | 16 | 2048 |
| | 13 | 512 | 32 | 16 | 32 | 4096 |
| | 14 | 512 | 32 | 16 | 32 | 4096 |
| | 19 | 64 | 16 | 2048 | 4096 | 4096 |
| | 21 | 64 | 16 | <16 | 2048 | 4096 |
| | 22 | 64 | 16 | 4096 | 4096 | 4096 |
| | 47 | 256 | 64 | 64 | 32 | 2048 |
| | 48 | 256 | 64 | 16 | 16 | 2048 |
| | 49 | 512 | 64 | 32 | 32 | 4096 |

7

```
     *   2 Vaccinations - 1st at 6 weeks of age
                         - 2nd at 9 weeks of age
     +   CPV Haemagglutination Inhibition Titres;  HIU/50uL
```

At six weeks of age (at time of first vaccination) puppy titres had fallen by 4 to 8 fold (< 16 to 64). 23 out of 33 (70%) pups had maternal antibody levels ≥ 16 HIU/50uL at six weeks of age.

After vaccination at six weeks of age 23 out of 24 (95.8%) pups responded to the vaccine containing CPV-2 P69 by eight weeks of age with titres in the range of 2048 to 16,384. Only two out of nine (22.2%) pups had responded in the same period to vaccination with Nobivac Parvo-C. Titres for Nobivac Parvo-C ranged from 2048 to 4096.

All pups, in both groups, which failed to respond to vaccination at six weeks of age responded to a second vaccination given at nine weeks of age. The magnitude of the response in these pups was equal to that of pups responding to the primary vaccination.

The serology results in Table 2 indicate that the attenuated live canine parvovirus P69 vaccine is highly immunogenic. 23 out of 24 pups with levels of passively acquired antibodies varying from < 16 to 64 produced an antibody response of at least 2048 HIU/50uL when vaccinated with a vaccine containing CPV-2 P69 at six weeks of age.

In contrast, only 3 out of 9 pups vaccinated at six weeks of age with Nobivac Parvo-C showed a rise in antibody titre. The three pups that showed an increase in antibody titre following the first vaccination all had prevaccination titres of 16. The remaining six pups had titres of 32 and 64, and no antibody response could be found.

The serology data shows that CPV-2 P69 overcomes levels of passively acquired antibodies in puppies that interfere with canine parvovirus vaccines existing in the prior art.

5A. Reversion to Virulence

In order to check for virulence or reversion to virulence of any virus material shed from vaccinated dogs, faecal samples from vaccinated dogs (passage 1) were collected and administered orally to previously CPV-naive dogs (passage 2). Further faecal samples from these latter dogs were taken and passaged through further unvaccinated dogs (refer Table 3).

Virus passaging was not observed except in two dogs Group 2B (refer Table 3) which developed detectable antibodies by day 14. However virus could not be detected in the faeces of 1B dogs nor in the faeces of 2B dogs. No other evidence of virus shedding could be found.

## TABLE 3

Reversion to Virulence Trial - Results

| Dog Passage No. | HI Titre (HIU/50 ul) | | |
|---|---|---|---|
| | Day 0 (PI) | Day 7 (PI) | Day 14 (PI) |
| Passage 1 | < 16, < 16 | 32768, 32768 | - |
| Passage 2 | < 16, < 16 | < 16, < 16 | - |
| Passage 3 | < 16, < 16 | < 16, < 16 | < 16, < 16 |
| Repeat Passage 2 | < 16, < 16 | < 16, < 16 | < 16, < 16 |
| Passage 1B | < 16, < 16 | 32768, 32768 | - |
| Passage 2B | < 16, < 16 | < 16, 1024 | 8192, 4096 |
| Passage 3B | < 16, < 16 | < 16, < 16 | < 16, < 16 |
| Passage 4B | < 16, < 16 | < 16, < 16 | < 16, < 16 |
| Passage 5B | < 16, < 16 | < 16, < 16 | < 16, < 16 |

2 dogs inoculated per passage.

Passage 1 dogs - vaccinated with 10 ml subcutaneously and 1 ml orally of P69 canine parvovirus.

Passage 2 dogs - administered 5 ml orally of a faecal suspension (equivalent to 2 grams of faeces) obtained from passage 1 dogs.

Passage 3 dogs - administered 10 ml orally of a faecal suspension (= 3 grams) obtained from passage 2 dogs.

Repeat Passage 2 dogs - Administered 10 ml orally of a faecal suspension (= 3 grams) (stored at -30$^{\circ}$C) from passage 1 dogs.

Passage 1B dogs - vaccinated with 10 ml subcutaneously and 1 ml orally of P69 canine parvovirus.

Passage 2B dogs - administered 10 ml orally of a faecal suspension (= 3 grams) obtained from Passage 1B dogs.

Passage 3B dogs - administered 30 ml faecal suspension (= 15 grams) obtained from Passage 2B dogs.

Passage 4B dogs - administered 30 ml of a faecal suspension (= 15 grams) obtained from passage 3B dogs.

Passage 5B dogs - administered 30ml of a faecal suspension (= 15 grams) obtained from passage 4B dogs.

5B. Comparison of Parvovirus Vaccine Strains in Seronegative Puppies

A comparison of the viral shedding characteristics and serology in puppies following administration of either CPV-2 P69 or CPV strain I-404 was carried out.

Pups of eight to eleven weeks of age were randomly segregated into four groups and inoculated with either CPV-2 P69 at a titre of $10^{6.0}$TCID$_{50}$ per dose or, CPV strain I-404 at the same dose, as shown in Table 4.

Serum antibody titres were measured before inoculation and at six and thirteen days after inoculation. Faecal samples were taken from each puppy on a daily basis beginning two days after inoculation for a total of twelve days. Each faecal sample was resuspended in Dulbeccos phosphate buffer (1:10), filtered to remove bacteria and inoculated into Feline Kidney cells.

TABLE 4

| SEROCONVERSION FOLLOWING INOCULATION WITH CANINE PARVOVIRUS STRAINS | | | | |
|---|---|---|---|---|
| Inoculum Group | Pup No. | CPV Haemagglutination Inhibition Titre (HIU/50uL) | | |
| | | DAY 0 | DAY 6 | DAY 13 |
| CPV-2 P69* | 08 | < 8 | 8192 | 8192 |
| (Subcutaneous) | 09 | < 8 | 8192 | 16384 |
| | 18 | < 8 | 2048 | 32768 |
| | 32 | < 8 | 1536 | 8192 |
| (group control) | 30 | < 8 | < 8 | < 8 |
| CPV Strain I-404* | 07 | < 8 | 4096 | 8192 |
| (Subcutaneous) | 12 | < 8 | 6144 | 16384 |
| | 17 | < 8 | 4096 | 6144 |
| | 19 | < 8 | 256 | 2048 |
| | 31 | < 8 | < 8 | 8192 |
| (group control) | 15 | < 8 | < 8 | < 8 |
| CPV-2 P69+ | 11 | < 8 | 384 | 16384 |
| (Oral) | 13 | < 8 | < 8 | < 8 |
| | 14 | < 8 | < 8 | ≧ 32768 |
| (group control) | 10 | < 8 | < 8 | Not tested |
| CPV Strain I-404+ | 16 | < 8 | 64 | 16384 |
| (Oral) | 33 | < 8 | 32 | 16384 |
| (group control) | 06 | < 8 | < 8 | < 8 |

\* Subcutaneous inoculation of $10^6$ TCID$_{50}$ per pup. Controls received sterile diluent.

+ Oral inoculation of $10^6$ TCID$_{50}$ per pup. Controls received sterile diluent.

Following subcutaneous inoculation 100% of pups receiving CPV-2 P69 had responded by 6 days after inoculation with titres of ≧ 1024 HIU/50uL. Whereas, only 60% (3 out of 5) of pups receiving CPV strain I-404 had responded by six days post inoculation with titres of ≧ 1024 HIU/50uL.

Following oral inoculation 1 out of 3 pups inoculated with CPV-2 P69 had seroconverted by six days post inoculation. By day 13 only 2 out of 3 had seroconverted. Both puppies administered CPV strain I-404 orally show seroconversion at 6 days post inoculation.

Canine parvovirus was detected in the faeces of all inoculated puppies as indicated in Table 5.

TABLE 5

| FAECAL SHEDDING OF VIRUS FOLLOWING INOCULATION | | | | | | | | | | | | | | |
|---|---|---|---|---|---|---|---|---|---|---|---|---|---|---|
| Inoculum Group No. | Pup | Virus Detected Post Inoculation (Days) | | | | | | | | | | | | |
| | | 2 | 3 | 4 | 5 | 6 | 7 | 8 | 9 | 10 | 11 | 12 | 13 |
| CPV-2 P69* | 8 | - | - | + | + | + | - | - | - | - | - | - | - |
| | 9 | - | + | + | + | + | - | - | - | - | - | - | - |
| | 18 | - | - | + | + | + | + | + | - | - | - | - | - |
| | 32 | - | - | + | + | + | - | - | - | - | - | - | - |
| (group control) | 30 | - | - | - | - | - | - | - | - | - | - | - | - |
| CPV Strain I-404* | 7 | - | - | - | + | + | + | - | - | - | - | - | - |
| | 12 | - | - | + | + | + | - | - | - | - | - | - | - |
| | 17 | - | + | + | + | + | + | + | + | - | - | - | - |
| | 19 | - | + | - | - | + | + | + | - | - | - | - | - |
| | 31 | - | - | - | - | + | + | + | + | - | - | - | - |
| (group control) | 15 | - | - | - | - | - | - | - | + | + | - | + | + |
| CPV-2 P69** | 11 | - | + | + | + | + | + | - | - | - | - | - | - |
| | 13 | - | - | + | - | + | - | - | - | - | - | - | - |
| | 14 | - | - | - | - | - | - | + | + | + | - | - | - |
| (group control) | 10 | - | - | - | - | - | - | - | - | - | - | - | - |
| CPV Strain I-404** | 16 | - | - | - | - | + | + | + | - | - | - | - | - |
| | 33 | - | - | - | + | + | + | - | - | - | - | - | - |
| (group control) | 6 | - | - | - | - | - | - | - | - | - | - | - | - |

* Subcutaneous inoculation of $10^6$ TCID$_{50}$ per pup.
** Oral inoculation of $10^6$ TCID$_{50}$ per pup.

Puppies inoculated with CPV-2 P69 shed virus earlier and for a shorter period than those puppies inoculated with CPV strain I-404. Also, CPV strain I-404 spread to the control in the subcutaneously inoculated group, whereas CPV-2 P69 did not infect any control.

6. Comparison of CPV Growth in Different Cell Cultures

CPV-2 P69 shows different characteristics to previously disclosed CPV strains when grown in various cell cultures.

In the present study, four separate cell lines were used for comparing the growth of CPV-2 P69. These were:

Madin Darby Canine Kidney (MDCK) cells, Crandell Feline Kidney (CrFK) cells, Feline Kidney (FK) cells, and A72 cells.

Duplicate flasks containing $5 \times 10^4$ cells/sq.cm. were inoculated with either CPV-2 P69 or Nobivac Parvo C batch (which is believed to include CPV Strain I-404) at an inoculum level of between $10^{4.7}$ - $10^{5.2}$ TCID$_{50}$.

Cell cultures were observed for cytopathic effect (CPE) following inoculation (refer Table 6A). When either virus caused 100% CPE in a cell type, then all flasks of that cell type were harvested by freezing and thawing. The harvest was titrated in CrFK cells (refer Table 6B and 6C). It is apparent from the results in Tables 6A-6C that CPV-2 P69 infects cell cultures and develops cytopathic effects (CPE) earlier than cultures infected with Nobivac Parvo-C the closest prior art. Differences in the yields of infectious virus from the different cell cultures were detected. The titres of both virus strains were lower when titrated on CrFK monolayer cultures, than when titrated in CrFK suspension cultures. The difference in titres between the two titration systems was greater for Nobivac Parvo-C. This difference between virus strains was highly significant ($p < 0.01$).

## TABLE 6A

| CYTOPATHIC EFFECT OF CPV STRAINS IN DIFFERENT CELL CULTURES | | | | | |
|---|---|---|---|---|---|
| Cell Types | Virus Strain | Hours Post Inoculation - CPE Score* | | | |
| | | 22 | 45 | 72 | 94 |
| FK | CPV-2 P69 | - | + | + + | + + + + |
| | Nobivac Parvo-C | - | + /- | + | + + |
| A72 | CPV-2 P69 | - | + | + + | + + + + |
| | Nobivac Parvo-C | - | + /- | + | + + |
| CrFK | CPV-2 P69 | - | - | + + | + + + + |
| | Nobivac Parvo-C | - | - | + | + + + |
| MDCK | CPV-2 P69 | - | - | - | + /- |
| | Nobivac Parvo-C | - | - | + /- | + /- |

\* Cytopathic effect (CPE) Score: - = No CPE
+/- = Questionable CPE
+ = Distinct CPE - limited extent
+ + = Obvious CPE - 50% of culture
+ + + = Obvious CPE - 75% of culture
+ + + + = 100% CPE

## TABLE 6B

| TITRE OF CPV STRAINS GROWN IN VARIOUS CELL CULTURES - TITRATION IN CrFK SUSPENSION CELL CULTURES | | | | |
|---|---|---|---|---|
| Virus Strain | Cell Types | | | |
| | MDCK | FK | CrFK | A72 |
| CPV-2 P69 | 4.65* | 4.8 | 4.65 | 5.5 |
| Nobivac Parvo-C | 4.55 | 5.65 | 5.0 | 5.65 |

*$TCID_{50}$ mL

## TABLE 6C

| TITRE OF CPV STRAIN GROWN IN VARIOUS CELL CULTURES - TITRATION IN CrFK MONOLAYER CELL CULTURES | | | | |
|---|---|---|---|---|
| Virus Strain | Cell Types | | | |
| | MDCK | FK | CrFK | A72 |
| CPV-2 P69 | 4.2* | 4.5 | 4.3 | 4.5 |
| Nobivac Parvo-C | 3.6 | 4.5 | 3.5 | 4.5 |

*$TCID_{50}$ mL

7. DNA Analysis

A feature of automonously replicating parvoviruses is that the genome contains a region that is duplicated and non-coding. This region of the genome is situated between nucleotides 4513 and 5011. This region of approximately 500 nucleotides is contained within a 1200 base HaeIII fragment. A proportion of this region between nucleotides 4465 and 5011 in the CPV genome has been sequenced for CPV-2 P69 and CPV-2 P98 as well as CPV strain I-404.

Base differences were located at approximately nucleotide 4600 as follows:

```
CPV-2 P69          TAT_CAACTA
CPV-2 P98          TAT_CAACTA
CPV Strain I-404   TCTTCAACTA
                   4595*       4604*
       *approximate nucleotide region
```

Further, with references to Fig. 1, a Hph-I digest of the CPV strain clearly showed that the third band in CPV-2 P69 was larger in molecular weight than the corresponding band in both CPV-2 P98 and CPV strain I-404 (Fig 1 - Std = LAMBDA BST EII).

**Claims**

1. An attenuated canine parvovirus having the antigenic and virulence characteristics of ECACC Accession No. V89042601 (CPV-2 P69)

2. An attenuated CPV as claimed in claim 1 further characterised in that any virus shed from animals innoculated with the attenuated CPV has a low rate of infection, as hereinbefore defined.

3. Canine parvovirus ECACC V89042601.

4. A vaccine comprising an effective amount of an attenuated canine parvovirus as claimed in any one of claims 1 to 3.

5. A vaccine as claimed in claim 4 in which the amount of CPV is at least $10^{4.8}$ 50% tissue culture infective doses ($TCID_{50}$) per dose.

6. A vaccine as claimed in any one of claims 4 or 5 in which the CPV is in either a live or attenuated form or both.

7. A vaccine as claimed in any one of claims 4 to 6 further comprising other pharmaceutically acceptable compounds, or any antigen or part thereof or virus or virus particle.

8. A vaccine as claimed in any one of claims 4 to 7 in the form of a lyophilised preparation or a suspension.

9. Use of an attenuated virus strain as claimed in any of claims 1 to 3, for the manufacture of a medicament for use in preventing infection by canine parvovirus in dogs.

10. Use according to claim 9, wherein the medicament is a vaccine as claimed in any of claims 4 to 6.

**Patentansprüche**

1. Attenuiertes canines Parvovirus mit den antigenen und Virulenzeigenschaften von ECACC Zugriffs-Nr. V89042601 (CPV-2 P69).

13

EP 0 485 463 B1

**2.** Attenuiertes CPV nach Anspruch 1, ferner dadurch gekennzeichnet, daß jedes Virus, das von Tieren abgegeben wird, die mit dem attenuierten CPV inokuliert sind, eine niedrige Infektionsrate aufweist, wie sie im vorhergehenden definiert ist.

**3.** Canines Parvovirus ECACC V89042601.

**4.** Vakzine umfassend eine wirksame Menge eines attenuierten caninen Parvovirus gemäß einem der Ansprüche 1 bis 3.

**5.** Vakzine nach Anspruch 4, in der die Menge an CPV mindestens $10^{4.8}$ 50 % Gewebekultur-infektiöse Dosen ($TCID_{50}$) pro Dosis beträgt.

**6.** Vakzine nach einem der Ansprüche 4 oder 5, in der das CPV entweder in einer lebenden oder attenuierten Form oder in beiden Formen vorliegt.

**7.** Vakzine nach einem der Ansprüche 4 bis 6, ferner umfassend andere pharmazeutisch verträgliche Verbindungen, oder jedwedes Antigen oder Teil davon oder Virus oder Viruspartikel.

**8.** Vakzine nach einem der Ansprüche 4 bis 7 in Form eines lyophilisierten Präparates oder einer Suspension.

**9.** Verwendung eines attenuierten Virusstammes gemäß einem der Ansprüche 1 bis 3 zur Herstellung eines Medikaments zur Verwendung bei der Prävention einer Infektion von Hunden mit caninem Parvovirus.

**10.** Verwendung nach Anspruch 9, wobei das Medikament eine Vakzine gemäß einem der Ansprüche 4 bis 6 ist.

**Revendications**

**1.** Parvovirus canin atténué ayant les caractéristiques antigéniques et de virulence de l'accès ECACC N° V89042601 (CPV-2 P69).

**2.** CPV atténué comme revendiqué à la revendication 1 caractérisé de plus en ce que tout virus disséminé d'animaux inoculés avec CPV atténué a un faible taux d'infection, comme défini précédemment.

**3.** Parvovirus canin ECACC V89042601.

**4.** Vaccin comprenant une quantité efficace d'un parvovirus canin atténué selon l'une quelconque des revendications 1 à 3.

**5.** Vaccin selon la revendication 4 dans lequel la quantité de CPV est d'au moins $10^{4,8}$ doses infectieuses à 50% de culture tissulaire. ($TCID_{50}$) par dose.

**6.** Vaccin selon l'une quelconque des revendications 4 ou 5 dans lequel le CPV est dans sous forme soit vivante soit atténuée soit les deux.

**7.** Vaccin selon l'une quelconque des revendications 4 à 6 comprenant de plus d'autres composés pharmaceutiquement acceptables, ou tout antigène ou partie de celui-ci ou virus ou particule de virus.

**8.** Vaccin selon l'une quelconque des revendications 4 à 7 sous la forme d'une préparation lyophilisée ou d'une suspension.

**9.** Utilisation d'une souche de virus atténué selon l'une quelconque des revendications 1 à 3 pour la fabrication d'un médicament pour une utilisation pour empêcher l'infection par le parvovirus canin dans les chiens.

14

**10.** Utilisation selon la revendication 9, dans laquelle le médicament est un vaccin comme revendiqué dans l'une quelconque des revendications 4 à 6.

*Std = LAMBDA BST E II